(19) Europäisches Patentamt

European Patent Office

Office européen des brevets

(11) Publication number: **0 458 653 A1**

## (12) EUROPEAN PATENT APPLICATION

(21) Application number: **91304747.8**

(22) Date of filing: **24.05.91**

(51) Int. Cl.⁵: **A61F 9/00**

(30) Priority: **24.05.90 US 527882**

(43) Date of publication of application:
**27.11.91 Bulletin 91/48**

(84) Designated Contracting States:
**DE ES FR GB IT**

(71) Applicant: **Pop, Mihai M.**
**108 Beausoleil**
**Gatineau, Quebec J8T 7H2 (CA)**

(72) Inventor: **Pop, Mihai M.**
**108 Beausoleil**
**Gatineau, Quebec J8T 7H2 (CA)**

(74) Representative: **Deans, Michael John Percy et al**
**Lloyd Wise, Tregear & CO. Norman House**
**105-109 Strand**
**London WC2R OAE (GB)**

(54) **Handtool for cataract surgery.**

(57) A handtool for use in emulsifying and aspirating a cataract from an eye has a cylindrical, motor-containing barrel to which irrigation and aspiration conduits are rigidly mounted. The motor drives a microdrill having a generally spherical head having dull teeth thereon. The drill shaft passes through an interior passageway connected to the aspiration conduit and the irrigation conduit is connected to an annular passageway concentric to the interior passageway. The drill head can be inserted into contact with the cataract through an opening in the capsule surrounding the lens and as it rotates it churns the cataract material into an emulsion which is aspirated, along with irrigation fluid from the vicinity of the rotating drill head.

FIG.1

EP 0 458 653 A1

The present invention relates to equipment for use in cataract surgery.

A cataract is, generally speaking, a disease of the eye in which the normally transparent lens becomes opaque. Treatment of this condition traditionally involves removal of the opaque lens (the cataract) and replacement thereof by an artificial lens implanted within the capsule that surrounds the lens.

In the past fifteen years the techniques and instrumentation for cataract extraction have evolved considerably. Starting with intracapsular extraction using a cryoprobe, which involves removal of the cataract and the surrounding capsule, the techniques have progressed to more modern extracapsular extraction using an irrigating aspirator, which involves a 12 mm to 15 mm slit in the eye, an opening in the capsule, and removal of the cataract therefrom, and further to phacoemulsification of the cataract using an ultrasonic vibrating tip that is inserted into the eye through a small slit of about 3 mm. The vibrating tip emulsifies the cataract so that it can be more easily aspirated. Recently, a laser pulsed device has been described for emulsification of the cataract through an even smaller incision but such devices are still in the developmental stage.

At the moment phacoemulsification is performed by only 20% to 30% of ophthalmologists in the United States and by fewer than that in Canada. The technique is difficult to master; there is a fairly high risk factor of damage from the vibrating tip; and the instrumentation needed for performing the surgery is very expensive ($40,000 to $70,000). For these reasons the technique is performed only on a limited basis, even though it has existed for over 10 years. There is therefore a distinct need for an inexpensive, yet effective, device which can be inserted into the eye to emulsify the cataract without major risk to the surrounding eye tissue.

In accordance with the present invention, I provide a handtool for use in surgically emulsifying and aspirating a cataract from an eye, characterised in that said handtool comprises: elongate housing means containing drive motor means connectable to a power source and to power control means, said motor means including a rotatable chuck portion at a forward end thereof; a shaft housing extending forwardly of said housing means, said shaft housing defining concentric interior and annular passageways extending therealong between proximal and open distal ends thereof; drill bit means having an elongate shaft receivable in said interior passageway, one end of said shaft being receivable in said chuck portion, the other end including a generally spherical head having a plurality of dull, generally radially projecting, teeth thereon; an irrigation conduit having one end connectable to a source of irrigation fluid and the other end connected to said shaft housing and communicating with said annular passageway; and an aspiration conduit having one end connectable to aspiration means and the other end connected to said shaft housing and communicating with said inner passageway.

The present invention operates on the simple principle of drilling.

I describe below a practical embodiment of my handtool which utilizes a round-tipped microdrill or microbit which can be inserted through a tiny slit in the eye, the slit being in the order of 2.1 mm. As the drill bit rotates it emulsifies the cataract that it contacts and the emulsified material is aspirated simultaneously through appropriate conduits positioned in close proximity to the bit. Preferably, the aspirating inlet and associated irrigation outlet are integrally provided in the nosepiece of the handtool.

The action on the cataract is at the tip of the bit which allows the ophthalmologist to bring the bit close to vital structure of the eye without a high risk of damage thereto. This possibility does not exist with phacoemulsification since the vibrating tip of the device, whether it be vibrated ultrasonically or by a pulsed laser, produces a shock wave that can damage surrounding structure in the eye during the emulsification process. While that shock wave is effective in emulsifying the cataract it can seriously damage the surrounding capsule or the iris if if contacts those tissues inadvertently.

The practical embodiment of my handtool described below has, for example, a variable speed 1.5 volt motor controllable by the surgeon. A removable nosepiece which terminates close to the bit tip includes conduit portions for irrigation fluid and aspiration of the fluid and the emulsified cataract. The surgeon needs only a small incision in the eye and he has a single-hand control of his instrument, requiring less dexterity and coordination than with prior techniques.

The invention is hereinafter more particularly described by way of example only with reference to the accompanying drawings, in which:-

Figure 1 shows in perspective an embodiment of handtool in accordance with the present invention in the form of a phacomicrodrilling device;

Figure 2 shows a partial longitudinal section of the device of Figure 1 illustrating the interior components;

Figure 3 is an enlarged perspective view of the tip area of the device of Figures 1 and 2;

Figure 4 is an enlarged cross-section of the drill bit as taken along the line 4-4 of Figure 3;

FIGURE 5 is an enlarged sectional view in the area where the irrigation and aspiration ports are located; and

FIGURE 6 is a simplified cross-section of the human eye showing the elements thereof of concern to the surgeon.

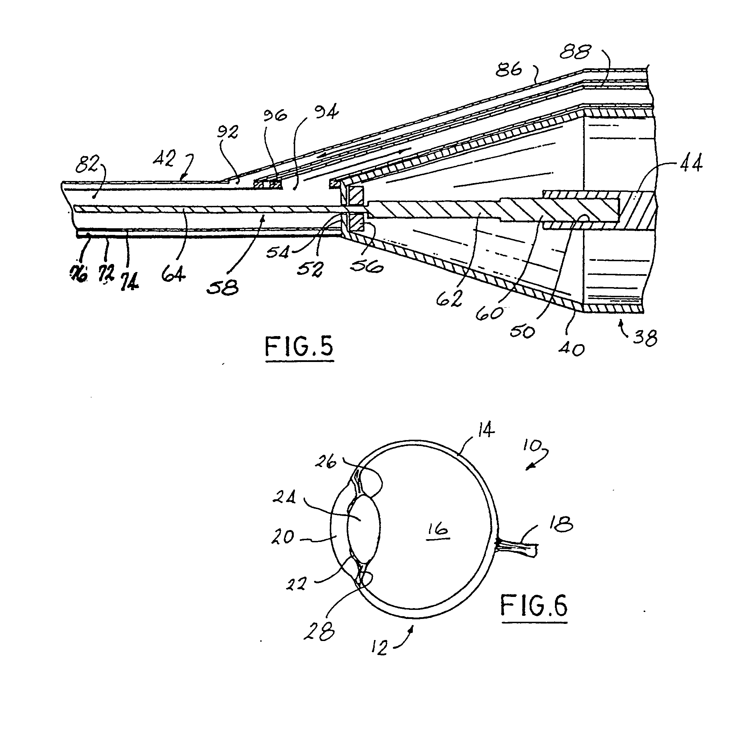

Figure 6 generally illustrates a human eye 10, the main body 12 including, although not separately shown, a plurality of layers 14, namely the sclera, choroid and retina. The eye also contains the vitreous body 16 (a fluid). The optic nerve 18 transmits a signal, received at the fovea centralis of the retina, to the brain. At the front of the eye is the cornea 20, iris 22 and the lens 24. The lens in a healthy eye is a transparent gelatinous material but in a diseased eye it becomes harder and opaque, causing deterioration of the individual's eyesight. The lens is contained within a thin capsule or shell 26 to which various muscles, such as are shown at 28, are connected. The muscles alter the configuration of the capsule 26 so as to alter the focal length of the lens during focusing of the eye. It is understood that an eye contains many more elements than are mentioned herein but Figure 6 shows all that is necessary for an understanding of the present invention.

With reference to the other drawings I provide a handtool 30 which is used to emulsify the cataract within the capsule 26 and to aspirate the emulsified material therefrom. The handtool 30 includes a small-diameter cylindrical housing or barrel portion 32 containing a variable speed electric motor 34. Preferably the motor has a rating of about 1.5 volts. It is connected to a suitable power source via a wire 36 and, preferably, there will also be provided a suitable power controller in the electrical circuit so that the surgeon can vary the speed of the motor 34 at will. The tool 30 also includes a separable nosepiece 38 which includes a frustoconical portion 40 leading from the diameter of the housing 32 down to that of a forwardly projecting shaft housing 42. Preferably the length of the tool 30 will be in the order of 15 cm while the diameter thereof will be in the order of 1.5 cm.

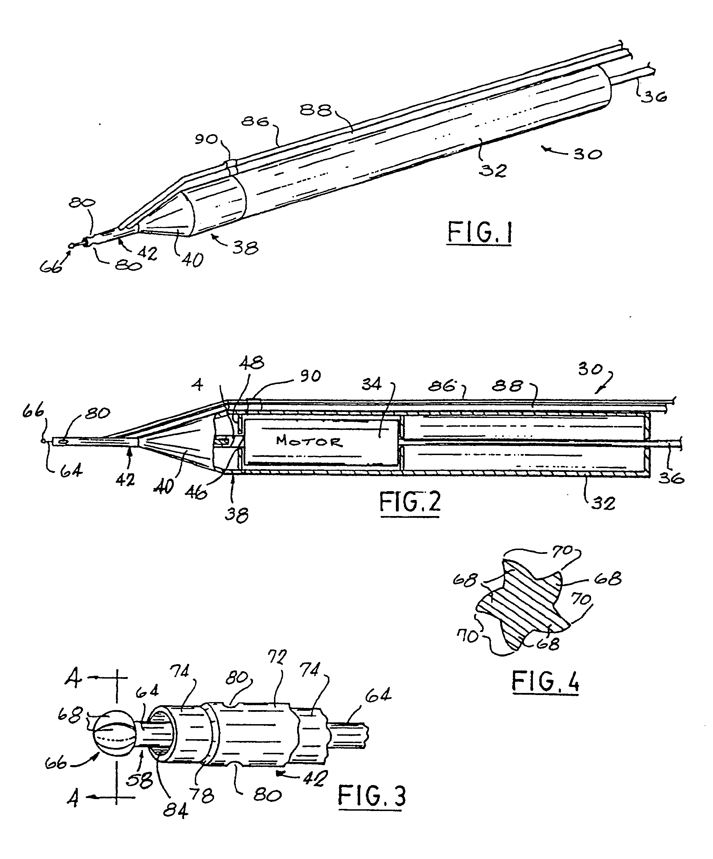

With reference to Figures 2 and 5 it will be seen that the motor 34 has a shaft 44 projecting forwardly therefrom through an opening 46 in the internal bulkhead 48 of the housing 32. The shaft 44 has a blind bore 50 emending axially from the front face thereof, the bore 50- serving as a chuck for the drill bit to be used .

At the juncture of the frustoconical section 40 and the shaft housing 42 there is another bulkhead 52 containing a central through opening 54. A replaceable resilient washer member 56 fits against the inside surface of the bulkhead 52 to help prevent the intrusion of unwanted fluids into the interior of the tool 30.

The bit to be used is a slightly modified version of a commercially available bit, not unlike a dentist's burr. The bit 58 has an enlarged shaft portion 60 at one end receivable in the bore or chuck 50 so that the bit can be driven by the motor. There is an intermediate diameter shaft portion 62 which leads from the portion 60 to the main shaft portion 64, the latter being extremely small in diameter. The intermediate portion stops just short of the washer 56 and prevents movement thereof away from the bulkhead 52. The head 66 of the bit is best seen in Figures 3 and 4 and it is evident that it has a generally spherical appearance with a plurality of curved teeth 68 extending radially from a central hub portion. The tips 70 of the teeth are somewhat dull to reduce damage that might otherwise occur if the teeth were sharp. The diameter of the bit head 66 will be less than the length of the incision to be made in the eye and thus will be in the order of 0.5 mm to 1.0 mm

The shaft housing 42 actually comprises a pair of concentric housing sections 72,74 best seen in Figures 3 and 5. The outer cylindrical housing section 72 extends from its proximal end adjacent the frustoconical portion 40 to its distal end just short of the end of the shaft housing 42. The inner cylindrical housing section 74 extends from its proximal end adjacent the frustoconical portion 40 to its distal end just beyond the outer housing section 72 creating an annular passageway 76 between the housing sections (Figure 5). That annular passageway is sealed at the outer end of the outer housing section 74 as at 78 although a plurality of radially directed circumferentially spaced openings 80 in the outer housing section 74 communicate the annular passageway 76 with the atmosphere.

The interior passageway 82 of the inner housing section 72 is open to the atmosphere at its outer end via an open end 84 through which the shaft 64 passes.

Irrigation and aspiration are achieved via independent conduits 86 and 88 respectively, which conduits are rigidly affixed to the tool 30 and which may be connected via flexible conduits (not shown) to respective irrigation and aspiration pumps as are known in the art. The conduits are separable as at 90 so that portions affixed to the removable nosepiece 30 are removable therewith from portions affixed to the main housing 32.

With particular reference to Figure 5 it will be seen that the irrigation conduit 86 communicates with the annular passageway 76 through an opening 92 and that the aspiration conduit 88 communicates with the interior passageway 92 of the inner housing section 74 via an opening 94. The opening 94 is sealed from the annular passageway 76 by an appropriate sealing member 96.

The technique of phacodrilling utilizing my handtool involves the surgeon making appropriately small incisions or openings in the cornea 20 and the capsule 26, using standard procedures, through the opening defined by the iris 22. An incision of about 2.1 mm is all that is required in the eye. The bit head 66 and the adjacent end of the shaft housing 42 are progressively inserted through the openings into the cataract with the head 66 being rotatably driven by the motor 34 under the surgeon's control. As the bit head 66 rotates it contacts the cataract and has the effect of slightly abrading the lens material that has hardened into the cataract. Irrigation fluid is introduced through the conduit 66, the annular passageway 76 and the openings 60 to the area adjacent the bit head 66 so as to emulsify the lens material that has been churned up by the head 66. Simultaneously, a vacuum is applied to the conduit 69 so that the emulsified lens material is aspirated, or sucked, from the capsule 26 through the opening 84, the interior passageway 82 of the inner shaft housing 74, and the conduit 99. The drilling procedure is continued until all of the diseased material has been removed from within the capsule 26. Thereafter the surgeon can complete the operation in the usual manner.

Tests on donated eyes have shown that by using my handtool and the drilling technique described it is possible to completely remove diseased lens material with little or no damage to surrounding tissue. The dull drill bit head 66 effectively works to emulsify the diseased lens material but it does not adversely affect the surrounding material, even when it accidentally comes into contact therewith. Accordingly a surgeon using the handtool is able to concentrate more on the task at hand rather than worrying excessively about damaging, perhaps irrevocably, the surrounding capsule, iris or cornea.

The desired handtool is not expensive to manufacture and the ancillary equipment, including the necessary pumps and electrical circuitry is readily available "off the shelf". A surgeon can set himself up to practice the phacodrilling technique with a small capital expenditure, with the expectation that many individuals requiring cataract surgery will request the technique in view of the inherent safety thereof.

The foregoing has described my handtool in the practical embodiment presently contemplated but it is clear that changes are feasible without altering the basic concept. For example, the motor 34 could be pneumatic rather than electric.

**Claims**

1. A handtool for use in surgically emulsifying and aspirating a cataract from an eye, characterised in that said handtool comprises: elongate housing means containing drive motor means connectable to a power source and to power control means, said motor means including a rotatable chuck portion at a forward end thereof; a shaft housing extending forwardly of said housing means, said shaft housing defining concentric interior and annular passageways extending therealong between proximal and open distal ends thereof; drill bit means having an elongate shaft receivable in said interior passageway, one end of said shaft being receivable in said chuck portion, the other end including a generally spherical head having a plurality of dull, generally radially projecting, teeth thereon; an irrigation conduit having one end connectable to a source of irrigation fluid and the other end connected to said shaft housing and communicating with said annular passageway; and an aspiration conduit having one end connectable to aspiration means and the other end connected to said shaft housing and communicating with said inner passageway.

2. A handtool according to Claim 1, further characterised in that said housing means includes an elongate cylindrical barrel portion containing said motor means and a nosepiece removable from said barrel portion.

3. A handtool according to Claim 2, further characterised in that said nosepiece includes a frustoconical section and said shaft housing, said shaft housing being of reduced diameter with respect to said barrel portion.

4. A handtool according to Claim 3, further characterised in that said nosepiece includes a transverse bulkhead at the juncture between said frustoconical seciton and said shaft housing, said bulkhead having an axial opening for passage of said shaft therethrough.

5. A handtool according to Claim 4, characterised in further including a sealing washer receivable on said shaft and positionable against said bulkhead within said furstoconical section.

6. A handtool according to any preceding claim, further characterised in that said shaft housing includes an inner cylindrical housing section defining said inertior passageway and a concentric outer cylindrical housing section defining said annular passageway with said inner housing section.

7. A handtool according to Claim 6, further characterised in that said annular passageway is sealed at the distal end thereof by sealing means extending between said outer and inner housing sections and a plurality of circumferentially spaced openings extend through said outer housing section adjacent the distal end thereof.

8. A handtool according to any preceding claim, further characterised in that said irrigation and aspiration conduits include separable portions rigidly attached to said barrel portion and to said nosepiece, respectively.

9. A handtool according to any preceding claim, further characterised in that said head has a diameter of 0.5 mm to 1.0 mm.

FIG.1

FIG.2

FIG.4

FIG.3

FIG.5

FIG.6

## European Patent Office

## EUROPEAN SEARCH REPORT

Application Number

EP 91 30 4747

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 436 091 (BANKO) <br> * abstract; figure 1 * | 1-4 | A61F9/00 |
| A | US-A-4 320 761 (HADDAD) <br> * abstract; figures 1-9 * | 1 | |
| A | US-A-3 809 093 (ABRAHAM) <br> * abstract; figures 1,7-12 * | 1 | |
| A | US-A-4 014 342 (STAUB ET AL.) <br> * abstract; figures 1-6 * | 1 | |
| A | US-A-4 167 944 (BANKO) | | |
| A | GB-A-2 093 353 (DYONICS INC.) | | |
| | | | TECHNICAL FIELDS SEARCHED (Int. Cl.5) <br><br> A61F <br> A61B |

The present search report has been drawn up for all claims

| Place of search | Date of completion of the search | Examiner |
|---|---|---|
| THE HAGUE | 30 JULY 1991 | SANCHEZ Y SANCHEZ J. |